# EUROPEAN PATENT APPLICATION

(11) **EP 3 797 826 A1**
(43) Date of publication of application: **31.03.2021**
(21) Application number: 19204316.4
(22) Date of filing: 21.10.2019
(51) Int. Cl.: A61N 1/372, H01Q 1/00

(54) **MEDICAL DEVICE**

(30) Priority: 27.09.2019 US 201962906738 P
(71) Applicant: BIOTRONIK SE & Co. KG, 12359 Berlin (DE)
(72) Inventor: Brown, James, Tigard, OR 97224 (US); Wang, Yu, Lake Oswego, OR 97035 (US); Austin, Eric, Portland, OR 97221 (US); Stadnik, Paul, Lake Oswego, OR 97035 (US)
(74) Representative: Galander, Marcus

(57) **Abstract**

A medical device (5) is disclosed, wherein the medical device (5) comprises a housing (10), wherein an electronic circuit of the medical device (5) is located inside the housing (10) and hermetically sealed therein from fluid intrusion, wherein the housing (10) comprises a front plate (11), a back plate and at least one side plate (12), wherein at least one of the front plate (11), the back plate and the at least one side plate (12) forms an interacting plate and comprises a first component (17, 37a, 37b, 47, 57a, 57b, 57c) comprising electrically conducting material and a second component (18, 38, 48, 58) comprising electrically insulating material, wherein the second component is accommodated within at least one through hole (19, 39, 49, 59) of the first component so that the first component is located at least partially around the second component, wherein the first component forms an active element of an antenna and is electrically connected to the electronic circuit of the medical device (5).

## Description

Medical devices for providing electrical stimulation to body tissues, for monitoring physiologic conditions, and for providing alternative treatments to drugs are well-known in the art. Many medical devices are implantable. Exemplary active implantable medical devices include implantable monitors, cardio defibrillators, pace makers, and programmable neuro-stimulator pulse generators. These medical devices typically incorporate a power source connected with an electronic circuit having a circuit board forming an electronic module. Connected to the sealed housing often a header assembly is provided which includes electrical contact elements that are electrically coupled with the electronic circuit and/or to the power source located inside the hermetically sealed device housing via a feedthrough component. The header assembly provides a connector means for electrically communicating via an external medical lead cable. The current implantable devices utilize a housing and a discrete feedthrough component to isolate the electronic circuit and the power source (battery) from body fluid intrusion.

Active medical devices often use telemetry systems for controlling or data exchange. The performance of radio frequency (RF) telemetry system of an active medical device is influenced by the proximity of the antenna to other metallic objects such as lead ports, outer wiring, etc. In one of the current designs where the antenna is located within the header, these other metallic objects are quite close to the antenna due to desired miniaturization of the header and co-located device housing feedthroughs. Additionally, this means that on the enclosed module, RF traces must be routed from the transceiver to the feedthrough. This type of design can lead to extra losses from the length of transmission line from the transceiver to the feedthrough, and requires extra shielding PCB layers and impedance matching components.

Document US 9,265,958 B2 describes an implantable medical device that includes a case having a conductive housing defining an opening. A dielectric material, e.g. a ceramic material, hermetically seals the opening of the housing. An antenna is positioned within the conductive housing behind the dielectric material. At least one dimension of the opening (e.g. length, width, or both) is larger than a corresponding dimension of the radiating element of the antenna. The implantable medical device according to this document still has a high number of components because the antenna is separate from the case.

Accordingly, there is a desire for an active medical device with an antenna which has an appropriate size and good radiation characteristics while decoupling the antenna from nearby metallic objects such as lead ports, recharge coils, and sense electrodes. Further, the performance of the RF system should be increased while simultaneously the length of RF transmission line needed on the device and the number of components, in particular the number of components located close to the feedthrough, is decreased.

The above task is solved by the following embodiments.

According to one embodiment the medical device comprises a housing, wherein an electronic circuit of the medical device is located inside the housing and hermetically sealed therein from fluid intrusion. Further, the housing comprises a front plate, a back plate and at least one side plate, wherein the front plate and the back plate are opposite plates of the housing with the largest surface. The at least one side plate connects the front plate and the back plate thereby forming a hollow housing body. At least one of the front plate, the back plate and the at least one side plate forms an interacting plate. The interacting plate comprises a first component of electrically conducting material and a second component of electrically insulating material, wherein the second component is accommodated within at least one through hole (aperture, opening) of the first component so that the first component is located at least partially around the second component, i.e. the first component forms a partial circumference or a full circumference of the second component. Additionally, the first component forms an active element of an antenna and is electrically connected to the electronic circuit of the medical device. As the active element of the antenna the first component receives and/or sends electromagnetic waves.

According to an embodiment of the present invention, the medical device comprises at least one more antenna construction as described above, comprising a first component and a second component, the first component forming an active element of an antenna. More than one antenna constructions can be implemented in the same front plate, back plate or side plate which forms an interacting plate. In an embodiment, more than one antenna constructions can be implemented in more than one of a front plate, back plate or side plate which form interacting plates. For the latter embodiment, the antenna construction with best transmission quality can be chosen, depending on the location and position of the medical device. Moreover, the antenna constructions can be used for different purposes, e.g. one for telemetry and one for wakeup function, operating in the same frequency range or in different frequency ranges.
According to the above embodiment, a plate is a three-dimensional structural object with a small thickness compared to its planar dimensions. The housing (can) of the medical device is composed of above plates which are connected to each other, for example by welding, in order to hermetically seal the interior of the housing from fluid intrusion. The at least one side plate may be bended or may form an edge. The electronic circuit is located within the interspace between the front plate and the back plate. In one embodiment, the front plate or the back plate is the interacting plate.

In one embodiment, the medical device is an implantable medical device, for example a neurostimulator, a defibrillator, a pacemaker, or an implantable monitor, and the electronic circuit is located inside the housing and hermetically sealed therein from body fluid intrusion. In a further embodiment the medical device comprises a header assembly and/or the antenna is an RF antenna.

The medical device according to the above embodiments has an improved RF performance due to mitigating the impact of non-RF device features on antenna radiation efficiency. In particular, according to the above embodiment, the antenna is located separate from the header assembly but integrated in the device housing as a hermetically-sealed aperture antenna. Accordingly, the design and manufacture of the header assembly is simplified and allows for smaller volume header structures. Hence, smaller devices due to fewer traces, including less RF grounding, are realized and lower component count on the module is achieved. Additionally, module layouts near the device housing feedthrough interconnect are simplified. Beside this, the location of the through hole or the second component may be defined such that it is in close proximity to an RF transceiver which is part of the electronic circuit accommodated within the housing. This simplifies the design of the electronic circuit or its module and reduces the length of the electrical connections from the first component to the electronic circuit.

According to an embodiment of the present invention, the medical device uses a frequency of 401-406 MHz, or 2.4-2.48 GHz for RF communication. According to an embodiment, the medical device uses a frequency of 2.4-2.48 GHz. According to an embodiment, the medical device uses a frequency of 800-1000MHz for RF communication.

The medical device may further comprise a header assembly providing at least one electric connector to at least one lead cable, to an electrode with sensing and/or stimulation function, or to a coil in the header, wherein the header assembly is attached to the housing and provides a feedthrough assembly forming the electrical connection of the at least one connector to the electronic circuit inside the housing. According to the above embodiment, the header assembly does not contain the antenna for telemetric application. Accordingly, fewer electrical connections to the header assembly at the feedthrough are needed.

The medical device may further comprise a power source accommodated within the housing. The power source, e.g. a battery, is electrically connected to the electronic circuit and hermetically sealed within the housing.

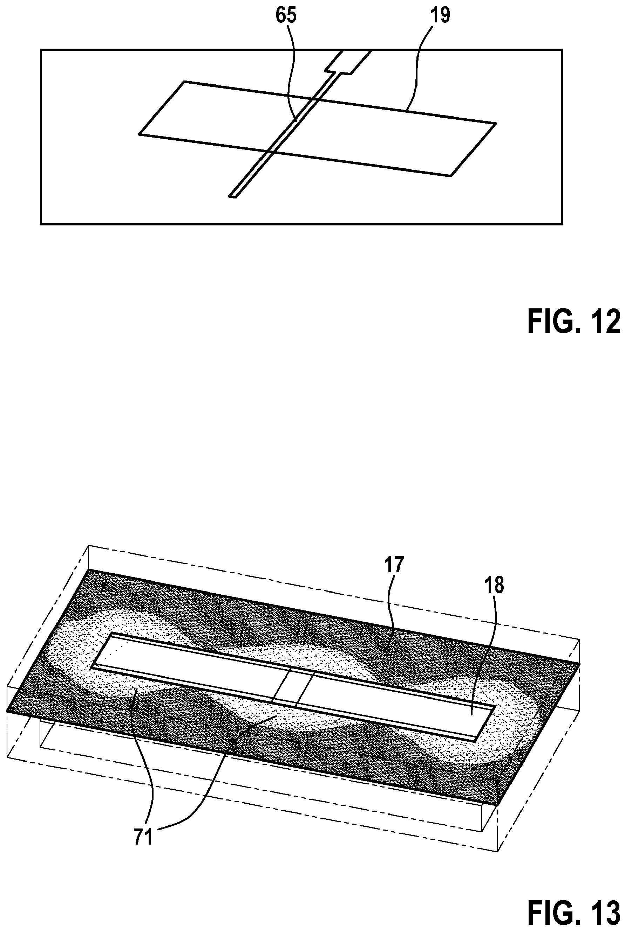

The size, shape and position of the at least one through hole of the first component (and thereby of the second component) can be varied in order to realize a particular band width and/or operating frequency of the antenna. In one embodiment the cross section of the at least one through hole of the first component and/or the cross section of the second component is a polygon (e.g. a rectangle or a square), a meander (rectangular meander, meander comprising rounded forms), an annular or elliptical ring, or at least two annular or elliptical rings located side by side with a through recess (in the case of through hole) or land (in the case of first component) connecting two of the rings or any combination of the preceding elements or similar forms. The meander may be defined as a combination of rectangular through holes. The cross section is, for example, the cross section along the main dimension (i.e. the planar dimension) of the interacting plate. Because the second component is located within the through hole in one embodiment the form and the size of a single piece second component corresponds to the form of the single through hole of the first component. This is an embodiment which may be realized with low production costs. Alternatively, there may be two or more through holes in a single-piece first component and accordingly a multi-piece second component. In a further alternative, the first component may comprise two or more parts. The first component and/or the second component may comprise a layered structure, for example comprising a protecting superstrate. The superstrate provides more consistent RF performance for the antenna based on the surrounding tissue type. As the dielectric properties of surrounding tissue may vary from patient to patient or based on the specific region of implantation, a more stable RF performance of the antenna (in terms of impedance and radiation efficiency) will ease in the development of the RF system. According to an embodiment of the present invention, the superstrate covers the portion of the can where the current is induced. Additionally, the superstrate serves some benefits related to smoothing over any potential edges from the aperture edges (ceramic feedthrough corners, flange corners, etc.). The superstrate may comprise at least one material of the group comprising epoxy, silicone, pellethane and polysulfone. The first component and the second component together form the interacting plate, e.g. the front or the back plate, wherein the outer surface of the interacting plate is flat. Alternatively, the outer surface of the interacting plate may be bent or curved. The interactive component of the antenna is formed by the first component, which is according to an embodiment of the invention the region of the edges around the second component. Induced currents for antenna communication occur on the edges. See Fig. 13 showing an embodiment of the invention, where the magnitude of the surface current density in the region of the edges around the second component. This means that the surface of the first component and of the second component does not form projections, in particular at the interface between the first component and the second component.

As indicated above, for a further enhancement of efficiency of the antenna, the dimensions of the at least one through hole (and thereby of the second component) and/or of the first component are adapted to the bandwidth and/or operating frequency of the antenna. For example, the dimensions of the antenna can vary according to the frequency of operation, and/or specific design issues. Antenna size can be kept small in order to fulfill geometry constraints of an implant. According to embodiments of the present invention, the length or width of the second component is 1 to 10 cm. According to a preferred embodiment of the present invention, the second component has a rectangular shape of 7.8 cm x 1.25 cm..

In another embodiment the first component is electrically connected to the electronic circuit by a ground pin and a signal pin or by a coaxial feed. This represents an embodiment which is easy to manufacture. According to an embodiment of the present invention, the medical device comprises a coaxial cable which is attached electrically connected to the electronic circuit at one end, and to the device housing on the other end. This embodiment would cause lower losses of transmission energy, facilitating to design a broadband impedance matching network for the antenna arrangement due to a more abrupt impedance transition.

In one embodiment the first component comprises or fully consists of electrically conducting material, wherein the electrically conducting material is at least one of the materials of the group comprising titanium, a titanium alloy, or any typical conductive material used for a housing of a medical device. According to an embodiment, the first component comprises a carbon fiber material. According to an embodiment, The first component comprises a coated plate. The plates of the housing which do not form the interacting plate may comprise or fully consist of the same material as the first component.

Further, the second component may fully consist of an electrically insulating material, wherein the electrically insulating material is at least one of the materials of the group comprising ceramic and glass, or any material that is used for electrical feedthroughs suitable for generating a hermetic seal. According to an embodiment of the invention, the second component comprises epoxy. According to an embodiment of the invention, the second component comprises an air gap.

A full and enabling disclosure of the present invention, including the best mode thereof, directed to one of ordinary skill in the art is set forth in the following specification. Thereby, further features and advantages are presented that are part of the present invention independently of the features mentioned in the claims.

The specification refers to the accompanying figures showing schematically
- Fig. 1: a first embodiment of an medical device in a perspective side view, partly transparent,
- Fig. 2: a cross section of the front plate (interacting plate) and a part of the interior of the housing of the embodiment of Fig. 1,
- Fig. 3: a model of a first embodiment of an interacting plate with a rectangular through hole in a perspective side view,
- Fig. 4: a representative radiation pattern of the model shown in Fig. 3,
- Fig. 5: a model of a second embodiment of an interacting plate in a top view,
- Fig. 6: a model of a third embodiment of an interacting plate in a top view,
- Fig. 7: a model of a fourth embodiment of an interacting plate in a top view,
- Fig. 8: a first embodiment of an electrical connection of the first component to the electronic circuit in a perspective view, partly transparent,
- Fig. 9: the electrical connection of Fig. 8 in a cross section,
- Fig. 10: a second embodiment of an electrical connection of the first component to the electronic circuit in a perspective view, partly transparent,
- Fig. 11: the electrical connection of Fig. 10 in a cross section, and
- Fig. 12: a microstrip transmission line stub feed below a rectangular through hole,
- Fig. 13: depiction of an embodiment, showing the magnitude of the surface current density in the region of the edges around the second component.

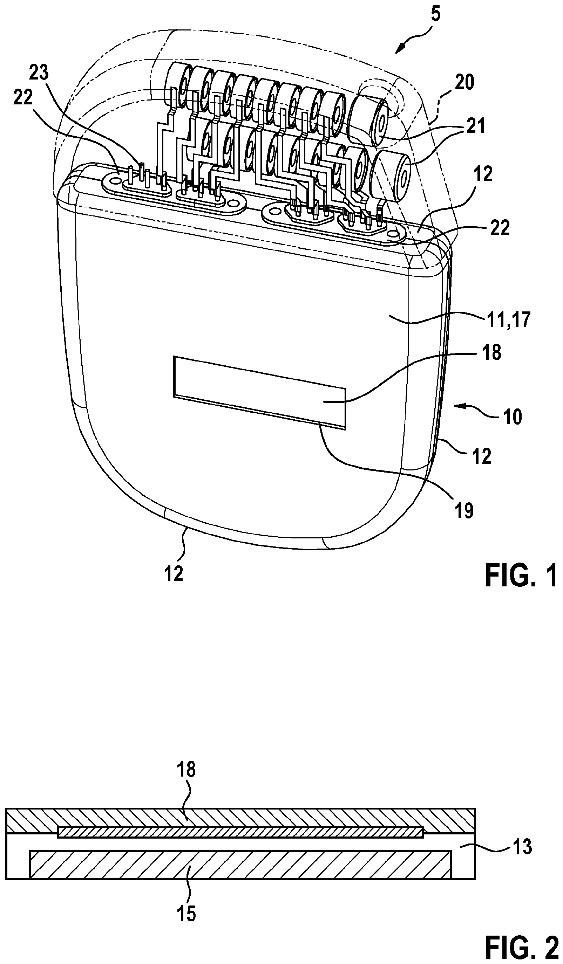

The first embodiment of a medical device 5 as depicted in Fig. 1, for example an implantable pace maker, comprises a housing 10 with a front plate 11, a back plate opposite the front plate 11 and several side plates 12 which are joined by seam welding. Further, the medical device 10 comprises a power source (not shown), e.g. a battery, and an electronic circuit (not shown) provided on a circuit board (e.g. printed circuit board, PCB) forming an electronic module 15 (see Fig. 2). The electronic circuit is electrically connected to the power source and comprises an RF transceiver for an RF antenna, both are part of the telemetry system of the medical device 5.

The medical device 5 further comprises a header assembly 20, partly shown transparent, including two connectors 21 for lead cables and two feedthrough assemblies 22 with lead ports 23 electrically connected to the respective terminal of the electronic circuit provided on the circuit board within the housing 10.

In the embodiment shown in Fig. 1 the front plate 11 forms the interacting plate. This plate consists of a first component 17 and a second component 18 located within a rectangular through hole (aperture, opening) 19 of the first component 18. The first component 17 consists of electrically conducting material and comprises, for example, titanium. The second component 18 is consists of electrically insulating material and comprises, for example, ceramic or glass. The second component hermetically seals the through hole 19 of the first component. The first component 17 surrounds the second component 18 and forms an active element of the antenna, i.e. the element receiving and/or sending electromagnetic waves. The antenna of the embodiment of Fig. 1 with its rectangular through hole 19 works analogous to a dipole antenna.

Fig. 2 shows a cross section of the embodiment shown in Fig. 1 within the through hole 19. This Fig. depicts the second component 18 forming a part of the front plate 11 (interacting plate) consisting of the electrically insulating material and the electronic module 15 comprising the electronic circuit with the RF transceiver. The interspace 13 between the second component 18 and the electronic module 15 may be filled with gas. The second component 18 may have a layered structure, for example, a ceramic or glass body and a protecting superstrate comprising epoxy.

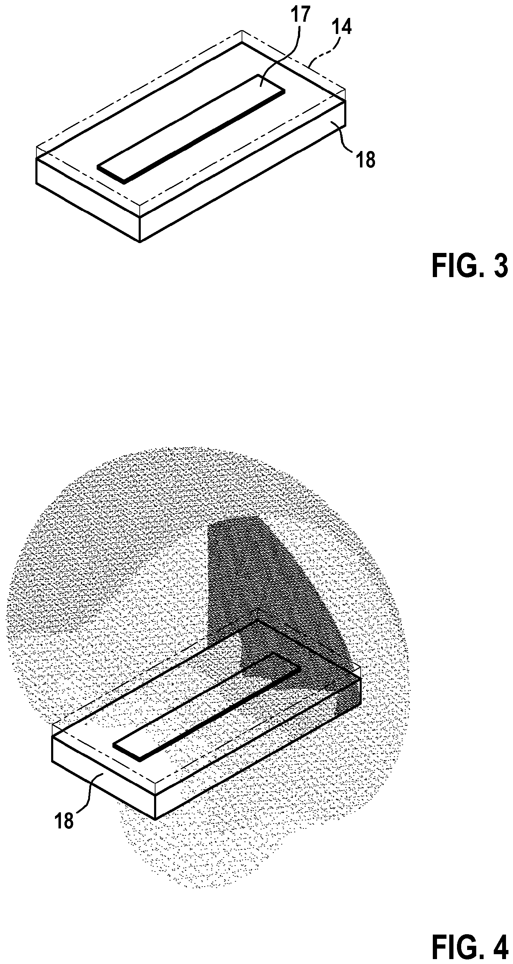

A rectangular model of the interactive plate of Fig. 1 is shown in Fig. 3 and 4, wherein the model comprises a protecting superstrate 14 covering the first component 17 and the second component 18. Protecting superstrate 14 acts as insulating material to the first component 17. Fig. 4 additionally shows a representative radiation pattern 30 for this particular model.

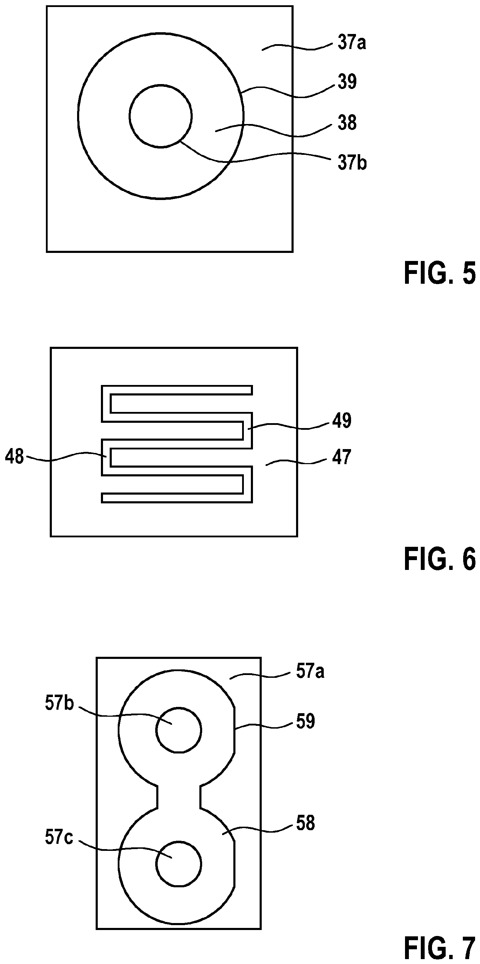

Fig. 5 to 7 depict alternative antenna designs with different cross section forms, wherein the cross section is meant along the planar dimension. The embodiment of an interactive plate of Fig. 5 comprises a through hole 39 and a second component 38 in the form of an annular ring. Accordingly, there is a two-piece first component 37a and 37b, wherein the first part 37a of the first component has a circular through hole and the cross section of the second part 37b of the first component forms a circle. In Fig. 6 the cross section of the through hole 49 of the first component 47 has the form of a rectangular meander. Accordingly, the cross section of the second component 48 has the same form. The embodiment of an interactive plate shown in Fig. 7 comprises a three-piece first component 57a, 57b and 57c. The cross section of the through hole 59 has the form of two annular rings connected by a longitudinal through recess. Accordingly, the cross section of the second component 58 has the form of two annular rings connected by a longitudinal land. Other forms and combinations of above forms are possible.

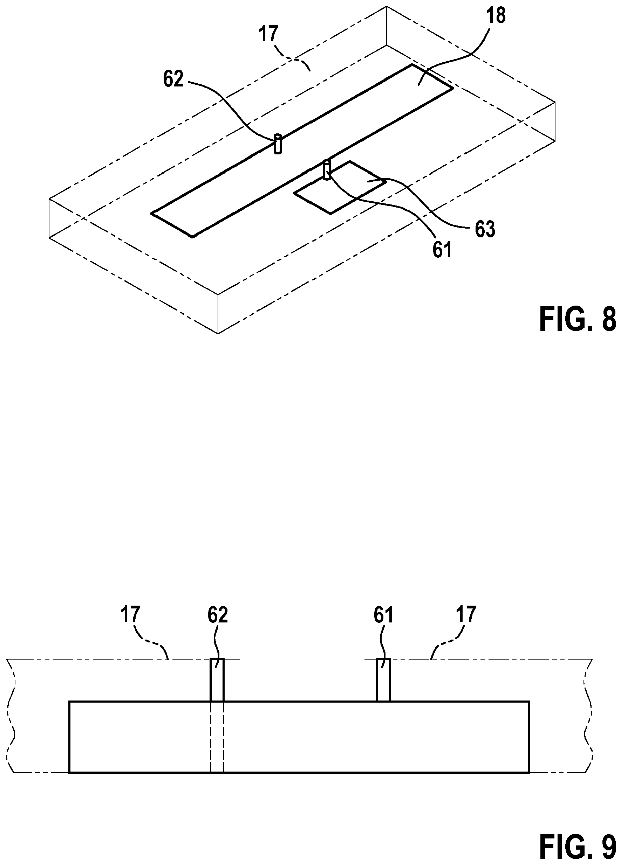

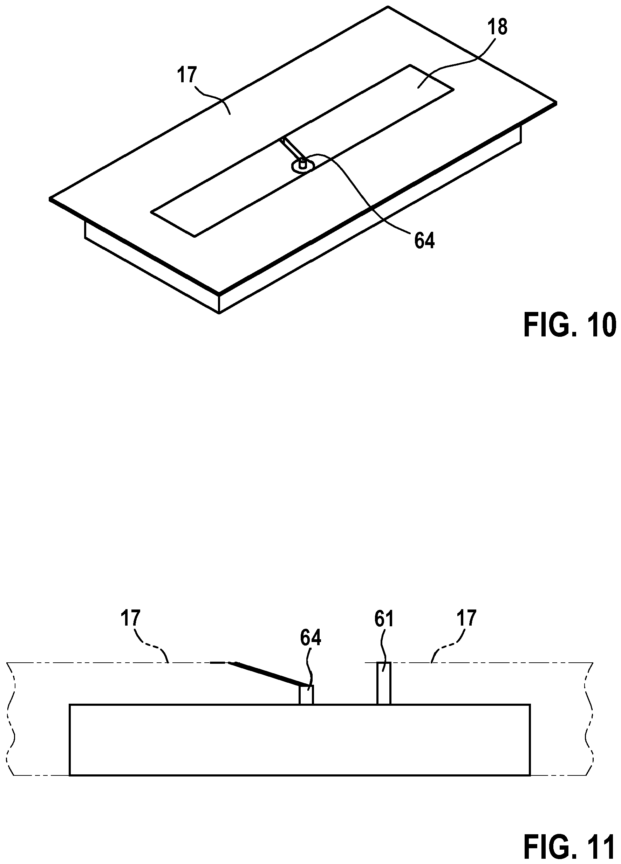

Fig. 8 to 12 disclose some embodiments for coupling RF energy between the active element of the antenna and the transceiver located at the electronic module 15 using direct (DC) connection or RF coupling capacitor. Fig. 8 and 9 show a first embodiment comprising a signal pin 61 and a ground pin 62. The signal pin 61 electrically connects one side of the first component 18 via the transmission line 63 to the transceiver (not shown) located at the electronic module 15. The ground pin 62 connects the opposite side of the first component 19 (with respect to the through hole 19) to the ground connection at the electronic module 15. The second embodiment depicted in Fig. 10 and 11 is similar to the embodiment shown in Fig. 8 and 9 but the signal pin is a coaxial feed 64 electrically connected to the first component 18 and the transceiver (not shown) at the electronic module 15. As indicated above, the active element of the antenna is located much closer to the RF transceiver thereby simplifying the module design. According to an embodiment, the signal pin 61 and/or ground pin 62 is capacitively coupled to the housing 10. The coupling should be configured such that the signal pin 61 and/or the ground pin 62 represent low impedances at RF frequencies. Moreover, it can be beneficial to choose capacitive coupling in order to protect the medical device from electrical effects as electrostatic discharge (ESD).

Fig. 12 shows a microstrip transmission line stub feed 65 below a rectangular through hole 19. Through hole 19 represents an embodiment of second component 18. Fig. 12 shows an extension of the embodiments in Figures 8 and 9, where transmission line 63 traverses the slot. In this embodiment, no shorting pins are used between the transmission line stub feed 65 and the housing, Alternatively, one shorting pin (not shown) can be used. Transmission line stub feed 65 can have a discontinuity in width as it traverses through hole 19, causing transmission line stub feed 65 to act as electrical stub. Width and length of the stub can be tuned such that the open circuit of the stub looks from an RF perspective like the short circuit, which would exist if a shorting pin was present. An advantage of this embodiment would be to reduce the number of direct connections to the feedthrough.

A tradeoff between the embodiment in Fig. 12 and those in Fig 8 and 9 may be based on the complexity of aperture/feedthrough fabrication and flexibility of impedance matching of the transceiver. Furthermore, a tradeoff between the placement of the components on the module and the optimal location of the aperture (i.e. second component 18, through hole 19) on the housing can be found.

Fig. 13 shows, according to an embodiment of the invention, first component 17, second component 18, and the magnitude of the surface current density 71 of the first component in the region of the edges around the second component. Current density 71 represents the induced current for antenna telemetry.

### List of reference signs

- 5: medical device
- 10: housing
- 11: front plate
- 12: side plate
- 13: interspace
- 14: superstrate
- 15: electronic module
- 17: first component
- 18, 38, 48, 58: second component
- 19, 39, 49, 59: through hole
- 20: header assembly
- 21: connector for lead cable
- 22: feedthrough assembly
- 23: lead port
- 30: radiation pattern
- 37a: first part of first component
- 37b: second part of first component
- 57a: first part of first component
- 57b: second part of first component
- 57c: third part of first component
- 61: signal pin
- 62: ground pin
- 63: transmission line
- 64: coaxial feed
- 71: current density

## Claims

1. A medical device (5) comprising
a housing (10), wherein an electronic circuit of the medical device is located inside the housing (10) and hermetically sealed therein from fluid intrusion,
wherein the housing (10) comprises a front plate (11), a back plate and at least one side plate (12), wherein at least one of the front plate (11), the back plate and the at least one side plate (12) forms an interacting plate and comprises a first component (17, 37a, 37b, 47, 57a, 57b, 57c) comprising electrically conducting material and a second component (18, 38, 48, 58) comprising electrically insulating material, wherein the second component is accommodated within at least one through hole (19, 39, 49, 59) of the first component so that the first component is located at least partially around the second component, wherein the first component forms an active element of an antenna and is electrically connected to the electronic circuit of the medical device (5).

2. The medical device of claim 1, wherein the medical device (5) is an implantable medical device and the electronic circuit is located inside the housing (10) and hermetically sealed therein from body fluid intrusion.

3. The medical device of any of the preceding claims, wherein cross section of the at least one through hole (19, 39, 49, 59) of the first component (17, 37a, 37b, 47, 57a, 57b, 57c) and/or the cross section of the second component (18, 38, 48, 58) is a polygon, a meander, an annular or elliptical ring, or at least two annular or elliptical rings located side by side with a through recess (in the case of through hole) or land (in the case of first component) connecting two of the rings or any combination of the preceding forms.

4. The medical device of any of the preceding claims, wherein the dimensions of the at least one through hole (19, 39, 49, 59) and/or of the first component (17, 37a, 37b, 47, 57a, 57b, 57c) are adapted to the desired bandwidth and/or operating frequency of the antenna.

5. The medical device of any of the claims, wherein the first component (17, 37a, 37b, 47, 57a, 57b, 57c) is electrically connected to the electronic circuit by a signal pin (61) and a ground pin (62).

6. The medical device of any of the claims 1 to 4, wherein the first component (17, 37a, 37b, 47, 57a, 57b, 57c) is electrically connected to the electronic circuit by a coaxial feed (64).

7. The medical device of any of the preceding claims, wherein the first component (17, 37a, 37b, 47, 57a, 57b, 57c) comprises or fully consists of electrically conducting material, wherein the electrically conducting material is at least one of the materials of the group comprising titanium, titanium alloy.

8. The medical device of any of the preceding claims, wherein the second component (18, 38, 48, 58) fully consists of an electrically insulating material, wherein the electrically insulating material is at least one of the materials of the group comprising.

9. The medical device of any of the preceding claims, wherein the antenna receives and/or sends electromagnetic RF waves.
